Europäisches Patentamt

European Patent Office

Office européen des brevets

(19)

(11) Numéro de publication : **0 130 916**
**B1**

(12) **FASCICULE DE BREVET EUROPÉEN**

(45) Date de publication du fascicule du brevet :
**11.11.87**

(21) Numéro de dépôt : **84401386.2**

(22) Date de dépôt : **29.06.84**

(51) Int. Cl.⁴ : **A 61 L 27/00**, C 23 C 22/73

(54) **Procédé pour réaliser des dépôts bioactifs de phosphates calciques et produits obtenus.**

(30) Priorité : **01.07.83 FR 8311026**

(43) Date de publication de la demande :
**09.01.85 Bulletin 85/02**

(45) Mention de la délivrance du brevet :
**11.11.87 Bulletin 87/46**

(84) Etats contractants désignés :
**BE CH DE GB IT LI NL**

(56) Documents cités :
**EP-A- 0 042 783**
**DE-A- 2 008 010**
**FR-A- 2 027 413**
**FR-A- 2 336 913**
**FR-A- 2 484 246**
**GB-A-   863 098**
**US-A- 3 918 100**

(73) Titulaire : **SOCIETE EUROPEENNE DE PROPULSION
(S.E.P.) Société Anonyme dite:
3, avenue du Général de Gaulle
F-92800 Puteaux (FR)**

(72) Inventeur : **Buttazzomi, Bernard Le Galion
23, Boulevard des Augustins Cieussa
F-13007 Marseille (FR)**
Inventeur : **Thebault, Jacques
100, Rue Etchenique
F-33200 Bordeaux (FR)**
Inventeur : **Constant, Georges
1, Rond point de la Palombière
F-31520 Ramonville St Agne (FR)**
Inventeur : **Leseur, Philippe
27 Résidence A Very
F-17000 La Rochelle (FR)**

(74) Mandataire : **Combe, André
CABINET BEAU DE LOMENIE 55, rue d'Amsterdam
F-75008 Paris (FR)**

**0 130 916**

## Description

La présente invention concerne un procédé pour réaliser des dépôts bioactifs de phosphates calciques ; elle concerne également les produits obtenus.

Dans la demande de brevet français 80 13 433 déposée le 17 juin 1980 et publiée sous le numéro 2.484.246 on a décrit un procédé pour la réalisation de revêtements bioactifs sur des prothèses osseuses, ce procédé étant caractérisé en ce que l'on réalise un brouillard de particules contenant des ions calcium et des ions orthophosphate, et on met ledit brouillard en contact avec un implant, en matériau inerte, dans des conditions telles qu'au moins un phosphate de calcium se forme par réaction sur la surface chaude dudit implant.

Dans ladite demande on a spécifié d'une part, que l'implant devait être chauffé à une température comprise entre 200 et 600 °C, et d'autre part que, pour augmenter la cinétique du dépôt (c'est-à-dire la vitesse de réalisation d'un dépôt d'une épaisseur convenable), il était souhaitable de préchauffer le brouillard en le faisant passer dans un four dont la température est de 100 à 300 °C. Dans les exemples de ladite demande le four de préchauffage était respectivement porté à 300 °C, 100 °C, 200 °C et 280 °C.

La poursuite des travaux sur la mise au point du procédé décrit dans la demande de brevet 80 13 433 a permis de déterminer les conditions optimales pour la réalisation des dépôts. Il est clair que la réalisation des dépôts selon les conditions expérimentales décrites, permet soit d'effectuer des dépôts selon la technique d'un dépôt en phase vapeur, soit selon la technique d'un dépôt en phase liquide ; plus précisément les dépôts (donc les phosphates formés sur le substrat) peuvent être constitués sur un substrat sec ou sur un substrat comportant un film d'eau. Les exemples fournis dans le brevet 80 13 433 semblent, pour des raisons liées à la vitesse de dépôt, privilégier la réalisation de ces dépôts sur un substrat sec ce qui impose une vaporisation complète (ou quasi complète) des gouttelettes de liquide formant le brouillard avant que lesdites gouttelettes atteignent la surface du substrat (ou implant).

Il a été trouvé maintenant et c'est l'objet de la présente demande que les dépôts effectués selon la technique du brouillard de particules liquides décrite dans le brevet 80 13 433, pouvaient très avantageusement être réalisés en phase liquide c'est-à-dire, en mettant en œuvre des conditions expérimentales qui font que, au cours du dépôt, il existe toujours un film liquide à la surface du substrat (ou implant).

Grâce à ce dépôt en phase liquide, il a été possible d'obtenir plus aisément, et avec une vitesse plus grande, des phosphates de calcium bioactifs permettant un rapport $Ca/_p$ supérieur à environ 1,1 (de préférence supérieur à 1,2) et de bonnes propriétés superficielles.

Dans le brevet anglais 863 098, on décrit un procédé pour réaliser un dépôt protecteur sur une surface métallique, ledit dépôt réagissant de préférence avec le métal de ladite surface, ledit procédé étant conduit de façon que chaque goutte de liquide déposée sur ladite surface sèche dans la position où elle heurte la surface. Dans ce brevet, on ne trouve pas d'enseignement impliquant la formation d'un film liquide sur la surface et on ne décrit pas la possibilité de réaliser un dépôt de phosphate de calcium.

Dans le brevet français 2.027.413 on trouve un enseignement très proche de celui du brevet anglais 863 098, en ce sens que les conditions du dépôt doivent être réglées de façon « que les particules de composition de revêtement déposées sur la surface restent à peu près sur leur point d'impact initial » (p. 6 lignes 32 à 34). De plus, ce brevet préconise l'utilisation d'une composition de fixation caractérisée en ce qu'elle comporte du chrome hexavalent.

La présente invention concerne un procédé pour la réalisation de revêtements bioactifs sur des prothèses osseuses, procédé dans lequel on réalise, à partir de solutions, un brouillard de particules contenant des ions calcium et des ions orthophosphate et on met ledit brouillard en contact avec un substrat tel qu'un implant, en matériau inerte, dans des conditions opératoires telles qu'au moins un phosphate de calcium souhaité se forme par réaction sur la surface chaude dudit substrat inerte, ledit procédé étant caractérisé en ce que les conditions opératoires pour effectuer le dépôt soient choisies de façon qu'il se forme un film liquide sur la surface chaude dudit substrat pendant le dépôt, la température dudit film liquide étant maintenue entre 55 et 90 °C.

Pour réaliser ce dépôt en phase liquide, il n'est plus souhaitable de préchauffer le brouillard de particules liquides et cela conduit à une simplification de l'appareillage utilisable. Dans la demande de brevet 80 13 433, on a décrit un appareil comportant un générateur d'ultrasons et une tubulure amenant dans un four les gouttelettes formées au niveau de l'appareil ultrasonore ; dans la présente application, la formation des gouttelettes peut être réalisée de façon classique par une pulvérisation pneumatique, grâce à laquelle lesdites gouttelettes sont projetées directement sur la surface du substrat à revêtir.

Une conséquence du non préchauffage des gouttelettes liquides et du fait que l'évaporation d'eau doit être effectuée à la surface du substrat, est que ledit substrat doit être chauffé énergiquement (autrement dit que l'on doit apporter audit substrat une quantité suffisante de calories par unité de temps). Il a été trouvé et cela constitue un autre aspect de la présente invention, qu'il était très avantageux de chauffer ledit substrat par un moyen de chauffage dans lequel les calories sont produites dans la masse dudit substrat ou dans une masse convenable au contact dudit substrat, comme par exemple les chauffages par infrarouge, par laser ou de préférence par induction haute fréquence.

Enfin, il a été trouvé que pour obtenir un dépôt de phosphate de calcium présentant une composition

2

(Ca/$_p$) et une cristallinité optimales, il était souhaitable que le film d'eau déposé sur le substrat, soit maintenu pendant la totalité du dépôt à une température comprise entre 55 °C et 90 °C.

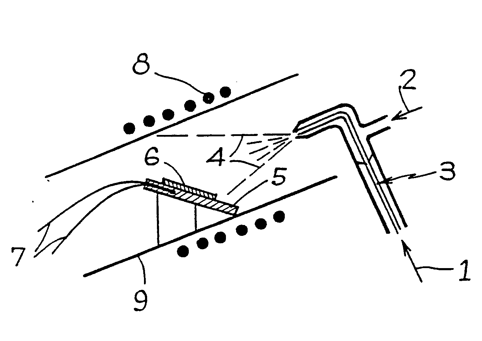

Le dispositif utilisé pour mettre en œuvre l'invention est illustré sur la figure unique ; cette figure montre

un appareil de pulvérisation pneumatique comportant un tube capillaire 3 par lequel arrive la solution 1 à pulvériser et une arrivée 2 du gaz vecteur (air comprimé) réalisant la pulvérisation.

un appareil de chauffage 8 formé de la boucle d'un four haute fréquence.

ce four chauffe un support en graphite 5 sur lequel est déposé le substrat 6a revêtu de phosphate ; la température du support 5 est contrôlée grâce à un thermocouple 7.

le jet 4 sortant du pulvérisateur pneumatique est projeté directement sur la surface du substrat 6.

Les exemples non limitatifs suivants illustrent l'invention (ces exemples visent à la réalisation de dépôts de phosphates de calcium de type hydroxyapatite) ;

## Exemple 1

On a réalisé, en utilisant le dispositif décrit ci-dessus, quatre dépôts de phosphate en maintenant à 260 °C, la température du support 5 (thermocouple 7). Les conditions expérimentales sont les suivantes :

Tableau I

| Dépôt | 1a | 1b | 1c | 1d |
|---|---|---|---|---|
| Débit de gaz (air) vecteur (l/min) | 7,3 | 8,2 | 9 | 9,8 |
| Film liquide sur le substrat | absent | épais | moyen | léger |
| Quantité de solution pulvérisée ml/h | 300 | 800 | 600 | 400 |
| Pression du gaz vecteur (kg/cm$^2$) | 1,5 | 2 | 2 | 2 |

Les dépôts obtenus ont été analysés pour déterminer notamment leur composition ; il a été trouvé que le rapport Ca/$_p$ était de 1,05 ± 0,05 dans les conditions 1a, de 1,25 ± 0,05 dans les conditions 1b, de 1,20 ± 0,02 dans les conditions 1c et 1d.

Il semble donc que quelle que soit l'épaisseur du film liquide, la composition des dépôts reste sensiblement la même, mais que ladite composition est nettement différente en absence du film liquide.

Des essais complémentaires ont montré que la qualité des dépôts obtenus avec le film liquide était supérieure à celle des dépôts obtenus en absence de film liquide.

## Exemple 2

Dans cet exemple on a cherché à déterminer l'importance de la température lors de la réalisation des dépôts de phosphate. Pour cela on a voulu tout d'abord trouver la relation qui existe, toutes choses égales par ailleurs, entre la température mesurée dans le support en graphite 5 (température mesurée par le thermocouple 7) et celle mesurée dans le film liquide.

Cette relation est la suivante

| Température dans 5 | Température dans le film liquide |
|---|---|
| 250 | 55 |
| 300 | 70 |
| 340 | 80 |
| 400 | 85 |
| 450 | 90 |
| 500 | 95 |

3

**0 130 916**

On a réalisé ensuite divers essais dans les conditions expérimentales suivantes (Tableau II)

Tableau II

| Dépôt | 2a | 2b | 2c | 2d | 2e | 2f | 2g |
|---|---|---|---|---|---|---|---|
| Température T ($^\circ$C)(en 5) | 260 | 300 | 330 | 360 | 380 | 400 | 500 |
| Débit de gaz (air) Vecteur (l/min) | 9 | 9,6 | 9,6 | 9,6 | 9,8 | 9,8 | 10,6 |
| Quantité de solution pulvérisée (ml/h) | 600 | 700 | 700 | 740 | 800 | 830 | 950 |
| Pression de gaz Vecteur (kg/cm$^2$) | 2,0 | 2,0 | 2,1 | 2,1 | 2,0 | 1,9 | 2,3 |

Pour tous ces essais on a pu constater que le dépôt était effectué avec une couche mince de liquide sur le substrat.

Les dépôts obtenus présentaient à l'analyse des valeurs de Ca/$_p$ d'environ :

2a = 1,20, 2b = 1,25, 2c = 1,28, 2d = 1,37, 2e = 1,27, 2f = 1,38, 2g = 1,35.

Les analyses par spectrométrie infrarouge ont permis de constater :

que le dépôt 2d, 2f et 2g avaient les caractéristiques des apatites déficientes en calcium.

que les autres dépôts semblaient constitués d'un mélange d'une phase apatitique déficiente en calcium et d'une phase phosphate bicalcique.

Tous les exemples ci-dessus ont été effectués en utilisant une solution aqueuse de départ contenant 120 mg de Ca H PO$_4$, 2 H$_2$O dans 1 litre d'eau distillée et en pulvérisant pneumatiquement cette solution avec, comme gaz vecteur, de l'air.

Les supports (ou substrats) revêtus des dépôts selon la présente demande se sont révélés particulièrement intéressants pour réaliser des prothèses osseuses (prothèses articulaires ou implants dentaires par exemple) du fait de leur propriété de se lier étroitement aux matériaux osseux avec lesquels lesdits dépôts sont mis en contact.

**Revendications**

1. Procédé pour la réalisation de revêtements bioactifs sur des prothèses osseuses, procédé dans lequel on réalise, à partir de solutions, un brouillard de particules contenant des ions calcium et des ions orthophosphate et on met ledit brouillard en contact avec un substrat tel qu'un implant, en matériau inerte, dans des conditions opératoires telles qu'au moins un phosphate de calcium souhaité se forme par réaction sur la surface chaude dudit substrat inerte, ledit procédé étant caractérisé en ce que les conditions opératoires pour effectuer le dépôt soient choisies de façon qu'il se forme un film liquide sur la surface chaude dudit substrat pendant le dépôt, la température dudit film liquide étant maintenue entre 55 et 90 °C.

2. Procédé selon la revendication 1, caractérisé en ce que pour maintenir la température dudit film liquide entre 55 et 90 °C on effectue le chauffage dudit substrat ou d'une masse convenable en contact direct avec ledit substrat par radiation infrarouge, par laser ou de préférence par induction haute fréquence.

3. Procédé selon l'une des revendications 1 et 2, caractérisé en ce que ledit brouillard de particules est réalisé par pulvérisation pneumatique et est projeté directement sur ledit substrat à revêtir.

**Claims**

1. Process for making bioactive coatings on osseous prostheses, said process consisting in forming, from solutions, a mist of particles containing calcium ions and orthophosphate ions, and in placing said mist in contact with a substrate such as an implant in inert material, in operational conditions such that at least one target calcium phosphate is formed by reaction on the hot surface of said inert substrate, said process being characterized in that the conditions of operation for making the deposit are so selected that a liquid film forms on the hot surface of said substrate during the deposition, the temperature of said liquid film being kept to between 55 and 90 °C.

2. Process according to claim 1, characterized in that, in order to keep the temperature of said liquid

4

film between 55 and 90 °C, said substrate or a suitable mass in direct contact with said substrate is heated by infrared radiation, by laser or preferably by high frequency induction.

3. Process according to one of claims 1 and 2, characterized in that said mist of particles is produced by pneumatic spray and is projected directly onto the substrate to be coated.

**Patentansprüche**

1. Verfahren zur Herstellung bioaktiver Ablagerungen auf Knochenprothesen, bei dem man aus Lösungen einen Nebel von Kalziumionen und Orthophosphationen enthaltenden Teilchen erzeugt und den Nebel in Kontakt mit einem Substrat, wie einem Implantat, aus inertem Material unter solchen Arbeitsbedingungen bringt, daß sich wenigstens ein gewünschtes Kalziumphosphat durch Reaktion auf der warmen Oberfläche des inerten Substrats bildet, dadurch gekennzeichnet, daß die Arbeitsbedingungen zur Durchführung der Ablagerung derart gewählt werden, daß sich ein flüssiger Film auf der warmen Oberfläche des Substrats während der Ablagerung bildet, wobei die Temperatur des flüssigen Films zwischen 55 und 90 °C gehalten wird.

2. Verfahren nach dem Anspruch 1, dadurch gekennzeichnet, daß man zum Halten der Temperatur des flüssigen Films zwischen 55 und 90 °C die Heizung des Substrats oder einer geeigneten Masse im direkten Kontakt mit dem Substrat durch Infrarotstrahlung, durch Laser oder vorzugsweise durch Hochfrequenzinduktion vornimmt.

3. Verfahren nach einem der Ansprüche 1 und 2, dadurch gekennzeichnet, daß der Teilchennebel durch pneumatische Zerstäubung erzeugt und direkt auf das zu überziehende Substrat gespritzt wird.